# FASCICULE DE BREVET EUROPEEN

(11) **EP 1 845 908 B1**
(45) Date de publication et mention de la délivrance du brevet: **07.01.2009**
(21) Numéro de dépôt: 06725971.3
(22) Date de dépôt: 09.02.2006
(51) Int. Cl.: A61F 5/01

(54) **Orthèse souple amovible pour atteinte du médio-pied**
Entfernbare flexible orthese für Mittelfussläsionen
Removable flexible orthosis for middle foot damage

(30) Priorité: 09.02.2005 FR 0501315
(43) Date de publication de la demande: 24.10.2007
(73) Titulaire: Shoukry, Kamel, 75013 Paris (FR)
(72) Inventeur: Shoukry, Kamel, 75013 Paris (FR)
(74) Mandataire: Hennion, Jean-Claude
(86) Numéro de dépôt international: PCT/FR2006/000299
(87) Numéro de publication internationale: WO 2006/085001

(56) Documents cités:
- WO-A-20/04017874
- FR-A- 2 606 630
- US-A- 4 271 605
- US-A- 4 313 433
- US-A- 4 597 395
- US-A- 5 330 419
- US-A- 5 620 413
- US-A- 5 755 679

## Description

La présente invention se rapporte de manière générale au domaine des orthèses. Plus particulièrement, l'invention a trait à une orthèse souple amovible pour atteinte ligamentaire , osseuse ou musculaire du médio-pied. S'agissant en particulier d'une entorse du médio-pied au niveau de l'articulation de Chopart et/ou de Lisfranc, cette orthèse vise à soulager la douleur, réduire l'oedème, à permettre la reprise immédiate de la marche et la cicatrisation de la plaie ligamentaire par le rapprochement des ligaments du médio-pied.

Les entorses de chevilles surviennent en France à raison de 6000 cas par jour. Parmi ces entorses, 20 à 25% sont des entorses du médio-pied qui passent le plus souvent inaperçues, cette pathologie n'étant pas ou peu connue et aucun traitement spécifique n'existant pour l'instant.

Parmi les traitements actuels proposés dans le cas de l'établissement d'un diagnostic d'entorse du médio-pied on peut citer la chaussure de Barouk, ou le bandage strapping sous forme de colliers métatarsiens, ou encore la botte plâtrée, qui tous soutiennent l'articulation du médio-pied, mais sans la maintenir en étau entre l'avant-pied et l'arrière-pied. De ce fait, la reprise de la marche est retardée. La disparition de la douleur ne survient qu'avec la botte plâtrée, et la marche se fait sans appui. Par ailleurs, l'utilisation de la botte plâtrée nécessite un traitement antiaggrégant plaquettaire jusqu'à déambulation complète et peut induire des troubles trophiques secondaires.

La mise en place de bandages élastiques (strapping ou taping selon l'élasticité des bandes) nécessite une technique adéquate et donc une formation appropriée, pour éviter un effet garrot avec oedème et points de compression douloureux qui apparaissent avec l'entorse. Les autres inconvénients des bandages élastiques, notamment du strapping, sont une possible intolérance cutanée, l'obligation de le renouveler environ tous les trois jours et surtout le fait que son renouvellement doit être effectué par du personnel spécialisé.

L'entorse du médio-pied est une des plus fréquentes et à ce jour il n'y a pas d'orthèse adaptée à son traitement. Le médio-pied est une zone de transition entre l'avant-pied, qui possède une structure horizontale, et l'arrière-pied, qui possède une structure verticale. Le médio-pied présente une structure frontale et instable, puisque multi articulaire, soutenue par une multiplicité de ligaments, soumise à des contraintes régulières (à chaque pas) et lourdes en poids (poids du corps).

Il existe plusieurs raisons qui rendent particulièrement difficile la réalisation d'une orthèse efficace et facile d'utilisation pour médio-pied:
- la constitution anatomique du médio-pied, comprenant l'articulation innominée, l'articulation médio-tarsienne de Chopart et l'articulation tarso-métatarsienne de Lisfranc ;
- l'approche clinique du médio-pied rendue difficile par son architecture particulière, sa complexité mécanique, ainsi que l'ampleur et l'importance des contraintes.

Le document US-4597395 décrit une orthèse dont la fonction est de comprimer les muscles, tendons et ligaments reliant la structure osseuse du tarse.

La présente invention a pour but de proposer une orthèse pour atteinte ligamentaire, osseuse ou musculaire du médio-pied, notamment pour entorse due à un étirement, à une rupture partielle ou totale des ligaments des articulations de Chopart et/ou de Lisfranc incluant les ligaments calcanéo-cuboïdiens, et le ligament bifurqué. Cette nouvelle orthèse est facile à utiliser par le patient et n'entraîne pas d'effets indésirables au porter.

A cet effet, l'invention a pour objet une orthèse souple amovible ayant une partie destinée à être appliquée sur l'arrière du pied et comportant :
- un premier collier métatarso-phalangien apte à être fixé sur l'avant-pied s'étendant, transversalement, des têtes du premier et du cinquième métatarsiens et, longitudinalement, depuis l'arrière de la racine des orteils vers la mi-diaphyse des métatarsiens et
- au moins une sangle de traction dont au moins une extrémité est fixée ou fixable sur le premier collier métatarso-phalangien, ladite sangle s'étendant entre le premier collier et la partie de l'orthèse destinée à être appliquée sur l'arrière du pied.

De manière caractéristique, l'orthèse selon l'invention assure le rapprochement de l'avant-pied et de l'arrière-pied en prenant en étau le médio-pied, ce qui maintient les éléments lésés en position de raccourcissement et de façon orientée. Cette action d'étau favorise la cicatrisation ligamentaire et ou osseuse et permet au pied de résister efficacement aux sollicitations extérieures, limitant considérablement le risque de récidive et rend au pied sa stabilité.

Dans une variante préférée de réalisation, l'orthèse selon l'invention comprend également un second collier malléolaire apte à être fixé autour de la cheville au niveau des malléoles interne et externe, de hauteur variable. Ce second collier est de préférence, au moins en partie, fixé à la sangle de traction, sur le bord supéro-postérieur de celle-ci.

D'autres éléments peuvent être ajoutés à l'orthèse selon l'invention, afin d'assurer la contention du pied de façon orientée, tels que, par exemple :
- une sangle de rappel élastique métatarso-fibulaire s'étendant entre le premier collier métatarso-phalangien et le second collier malléolaire, et
- un collier du tarse, destiné à recouvrir les faces interne, inférieure et externe du médio-pied, la sangle de rappel métatarso-fibulaire et à se refermer sur lui-même en s'appliquant partiellement sur le second collier malléolaire et sur lui-même par auto-grippant.

Ce collier du tarse peut être fixé à la sangle de rappel ou sur le bord supérieur et interne de la sangle de traction venant alors recouvrir le médio-pied et la sangle de rappel pour plaquer celle-ci contre le médio-pied.

D'autres caractéristiques et avantages de l'orthèse de la présente invention apparaîtront à la lecture de la description détaillée qui va suivre d'exemples de réalisation, donnée en référence aux dessins annexés non limitatifs de l'invention, dans lesquels :

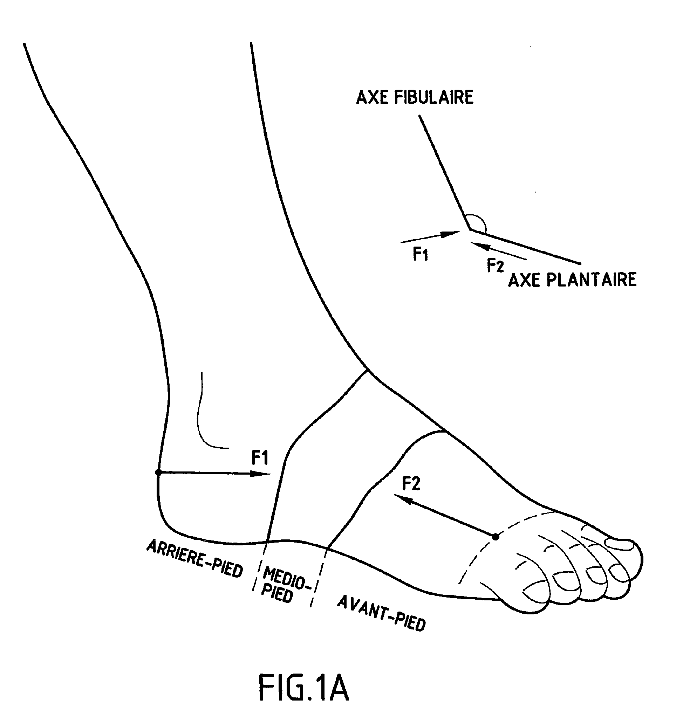

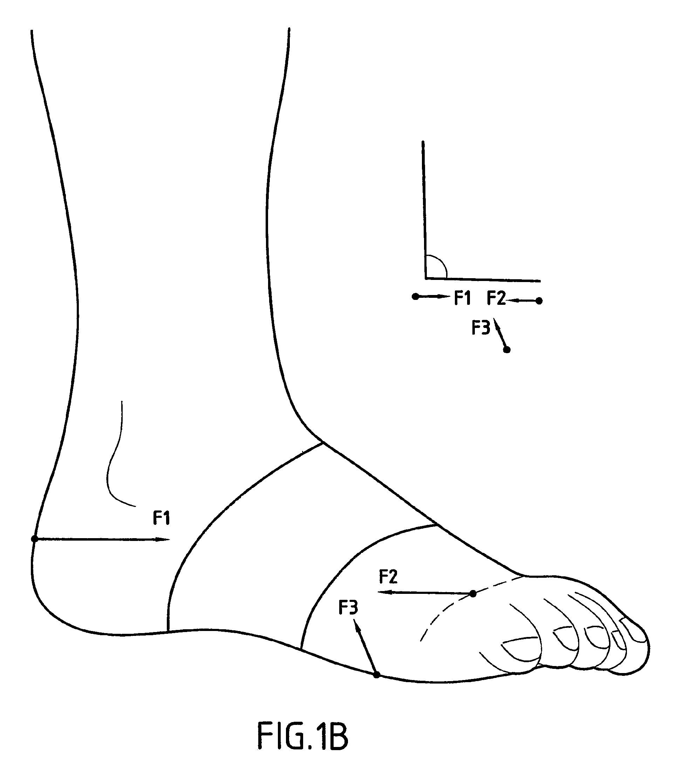

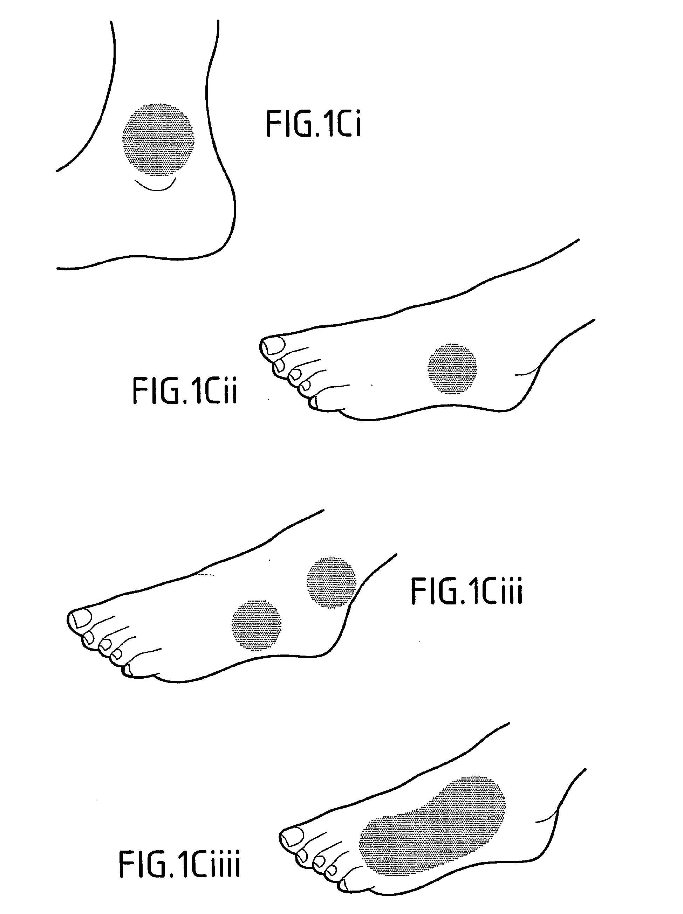

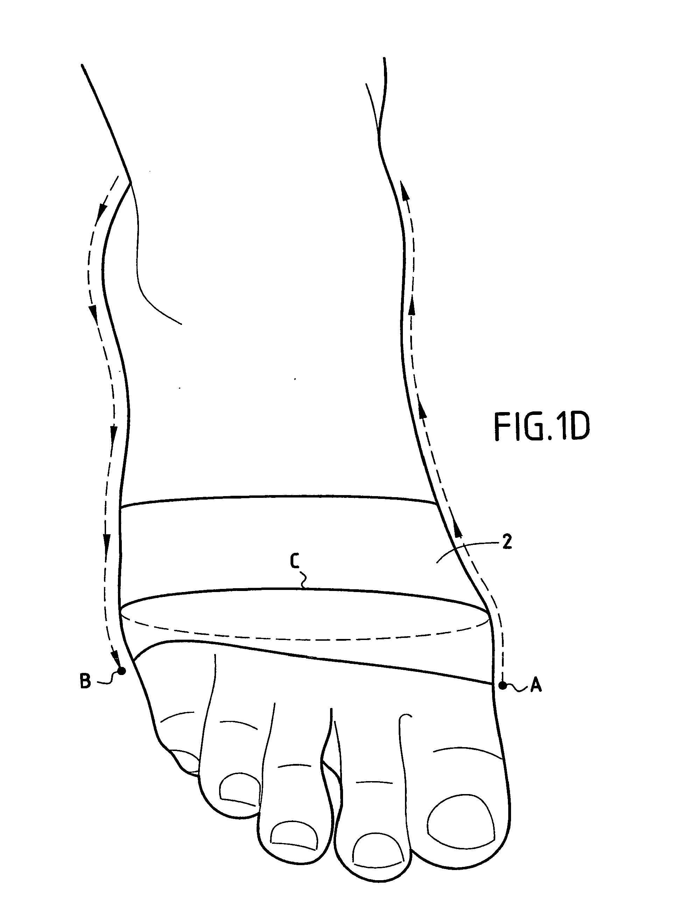

- la figure 1 représente de manière schématique un pied vu de trois-quarts, en visualisant les forces F1 et F2 s'exerçant sur le médio-pied lors du port de l'orthèse de l'invention ; on note que le pied au repos forme un angle obtus avec la jambe (axe fibulaire et axe plantaire) ;
- la figure 1b montre la position de la force F3 qui ramène le pied en angle droit par rapport à la jambe ;
- la figure 1c montre la position des hématomes sur la face externe de la cheville et du pied indiquant la présence de lésions ligamentaires ou osseuses au niveau d'une ou plusieurs articulations :
   o l'hématome de l'entorse de cheville (i) situé sur et autour de la malléole externe,
   o celui de l'entorse du Chopart (ii) situé en avant de la dépression pré-malléolaire sur le médio-pied,
   o la double entorse de Chopart et de cheville (iii), et
   o l'entorse de Lisfranc (iiii) où l'hématome recouvre l'avant du médio-pied et les métatarsiens ;
- la figure 1d montre un pied vu d'un angle supéro-antérieur, montrant la ligne en pointillé AB qui représente la pointure du pied mesurée en centimètres, et la circonférence C de la tête des métatarsiens à son point le plus large ;
- les figures 2a et 2b représentent un premier exemple de réalisation de l'orthèse de l'invention, comportant le premier collier métatarso-phalangien, la sangle de traction, et le second collier malléolaire, positionnée sur un pied, vu de profil externe pour la figure 2a et vu de profil interne pour la figure 2b ;
- les figures 3a à 3d illustrent l'orthèse de l'invention selon un second exemple de réalisation comportant de plus une sangle de rappel et un troisième collier du tarse sur un pied, vu de profil externe pour la figure 3a, vu de la face inférieure pour la figure 3b, vu de profil externe avec le troisième collier du tarse rabattu pour la figure 3c et vu de profil interne pour la figure 3d ;
- Les figures 4 illustrent , vu du profil externe du pied, une autre variante de l'orthèse de la figure 3 avec une sangle de rappel trapézoïdale dont l'extrémité inférieure est fixée sur le collier métatarso-phalangien et sur la sangle de traction et dont l'extrémité supérieure fait le tour de la cheville sur sa face postérieure, étant logée dans une ganse, pour se fixer en avant du collier malléolaire ;
- la figure 5 illustre, vu de profil interne du pied, une orthèse dans laquelle le troisième collier malléolaire est fixé sur le bord interne de la sangle de traction ;

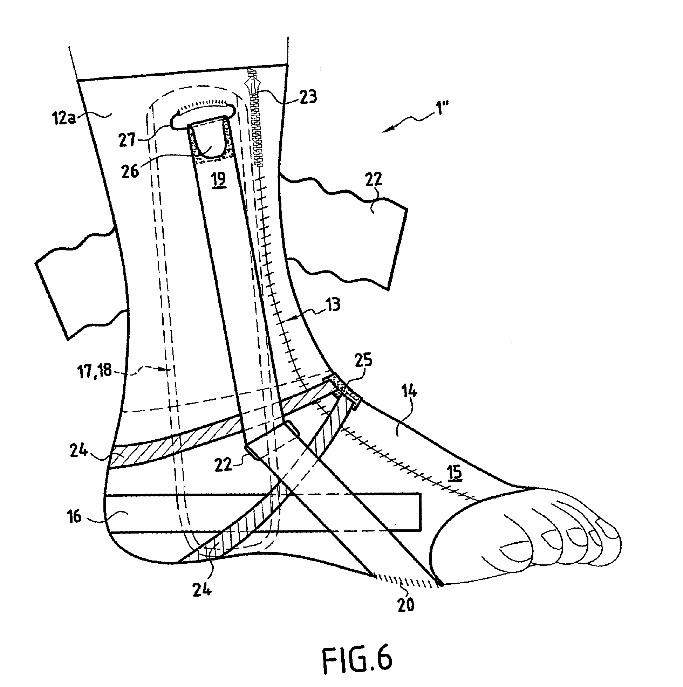

- la figure 6 illustre un exemple de réalisation de l'orthèse de l'invention présentant une structure tricotée en forme de chaussette.

L'orthèse selon l'invention a pour fonction principale le rapprochement de l'avant-pied et de l'arrière-pied en prenant le médio-pied en étau, comme représenté dans la figure 1 annexée, sous l'effet des forces F2 et F1. Cette action permet de maintenir les éléments lésés en position de raccourcissement et de façon orientée. Les éléments lésés se situent sous les hématomes, qui indiquent, de manière indirecte, la localisation de la lésion (figure 1c). La force F3 ramène la semelle du pied (axe plantaire) à angle droit par rapport à l'axe de la jambe (axe fibulaire). Elle raccourcit le trajet des muscles péroniers, ce qui a pour effet de faire disparaître la contracture et par conséquent la douleur.

Cette contention orientée suivant la reconstitution de l'anatomie concerne les articulations respectives de Chopart et de Lisfranc. Elle est obtenue au moyen d'un ensemble de sangles et de colliers assemblés entre eux et judicieusement orientés pour assurer les différentes lignes de forces.

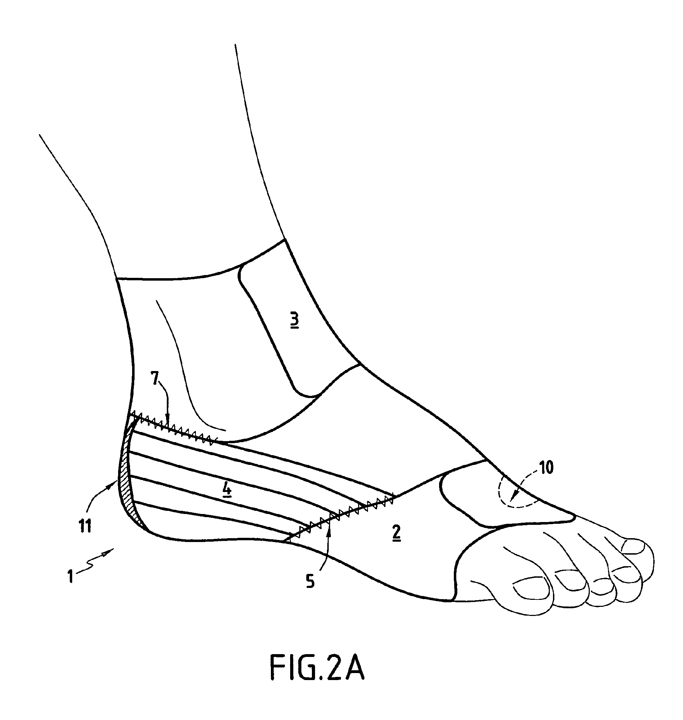

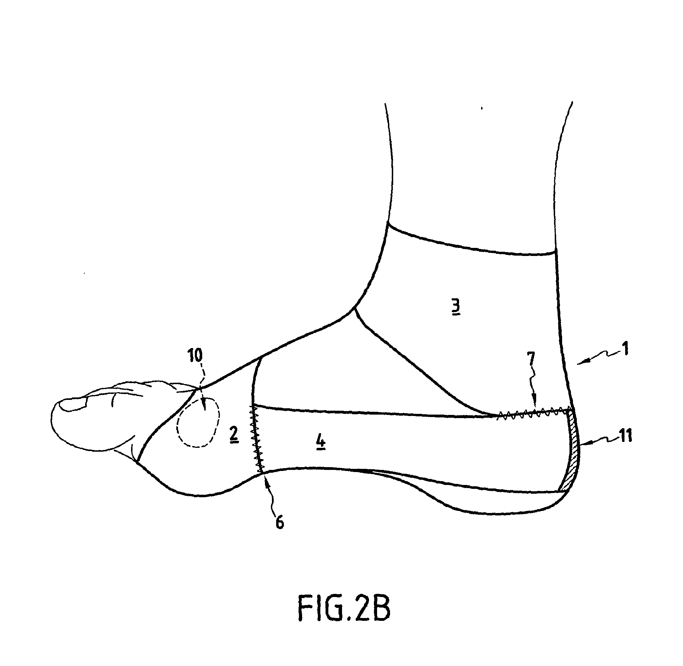

Selon le premier exemple de réalisation illustré aux figures 2a et 2b, l'orthèse 1, est spécialement adaptée au traitement des atteintes ligamentaires du médio-pied externe. Elle comprend un premier collier métatarso-phalangien annulaire réglable 2 servant de fixateur antérieur et une sangle métatarso-calcanéo-métatarsienne de traction 4 en forme d'étrier, la combinaison de de premier collier et de cette sangle de traction permettant d'exercer l'effet de traction nécessaire à la mise en étau du médio-pied entre l'arrière-pied et l'avant-pied. Le premier collier métatarso-phalangien 2 recouvre l'avant-pied en s'étendant, transversalement, entre le pied des têtes des premier et cinquième métatarsiens et, longitudinalement, de l'arrière de la base des orteils vers la mi-diaphyse des métatarsiens. Ce premier collier 2 a une largeur (dimension prise dans la direction longitudinale de l'avant-pied) qui peut être de 5 à 10 centimètres selon la taille de l'individu. Il recouvre et enserre au moins la zone circonférentielle C (figure 1D) entre les têtes proéminentes du premier et du cinquième métatarsien, qui est la zone la plus large du pied, et la zone qui est en avant de cette zone circonférentielle C, dans la direction des orteils. Ainsi le premier collier métatarso-phalangien 2 est parfaitement bloqué en position lors de l'application de la force (F1) exercée par la sangle de traction 4. Sur la figure 1D, ce premier collier 2 s'étend de la racine des orteils à mi-longueur des diaphyses métatarsiennes.

La sangle de traction 4 a dans le premier exemple, la forme d'un étrier, intégrant la partie de l'orthèse qui est destinée à être appliquée sur l'arrière du pied et délimitant de part et d'autre de cette partie arrière une sangle de traction interne et une sangle de traction externe. L'invention n'est cependant pas limitée à ce mode de réalisation, la partie arrière pouvant être indépendante de la sangle de traction, celle-ci pouvant être uniquement interne ou externe.

La partie arrière de l'orthèse, qui, dans le premier exemple, est la partie coudée de la sangle de traction 4, est appliquée sur la zone du calcanéum qui constitue, avec la zone circonférentielle C ci-dessus, la seconde zone d'ancrage de l'orthèse permettant l'application des forces F1 et F2 et réalisant la prise en étau du médio-pied entre l'avant-pied et l'arrière-pied. C'est à ce titre que la sangle de traction 4 peut être dénommée métatarso-calcanéo-métatarsienne.

Il est préférable d'éviter l'application de la partie arrière de l'orthèse sur la zone du tendon d'Achille, susceptible de constituer une gêne, voire un traumatisme localisé, du fait des forces mises en jeu.

Les deux extrémités de la sangle de traction 4, en forme d'étrier, sont rendues solidaires du premier collier 2 par deux lignes de couture 5 et 6 pratiquées de façon symétrique selon les bords postéro-interne et postéro-externe dudit premier collier 2.

De manière préférée, l'orthèse 1 comprend un second collier malléolaire annulaire réglable 3, servant de fixateur au niveau de la cheville.

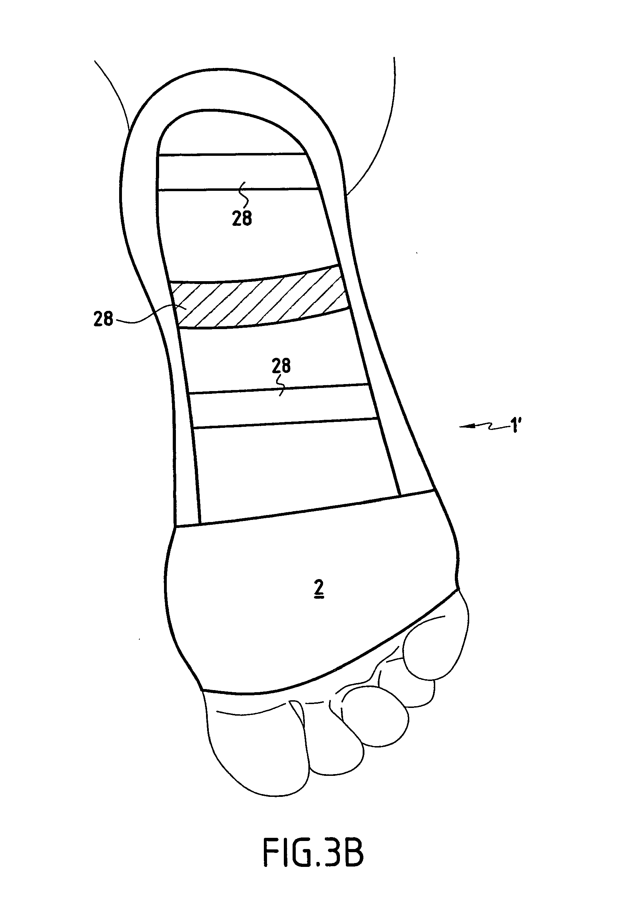

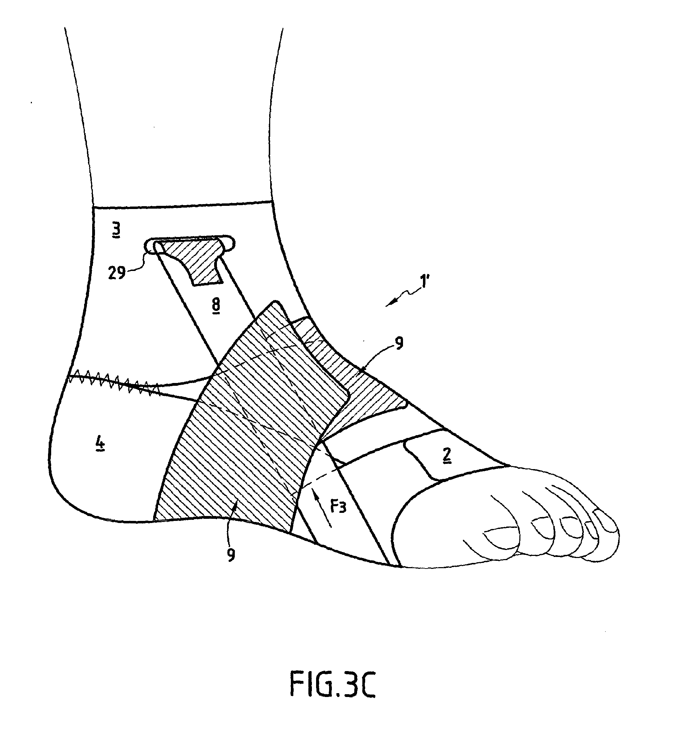

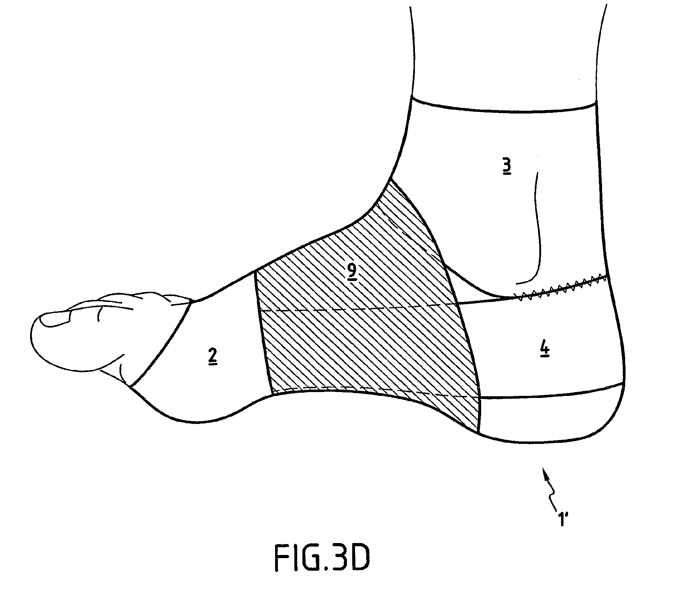

Dans sa partie arrière, la sangle de traction 4 est fixée par une couture partielle 7 à un secteur limité de la lisière inférieure du second collier malléolaire 3. Attachée de part et d'autre de la sangle de traction 4 peut être prévue une ou plusieurs bandelettes 28, située sur la face plantaire du pied (visible sur la figure 3b). Cette bandelette 28 sert à empêcher l'extrusion du pied de l'orthèse vers le bas, suite à un mauvais chaussage de l'orthèse.

Bien entendu, il existe plusieurs longueurs de sangles de traction 4 en fonction des familles de pointures retenues. Le réglage en circonférence du premier collier métatarso-phalagien 2 est réalisé de préférence par auto-agrippant. Comme indiqué précédemment, la zone circonférentielle métatarsienne C (figure 1d, ligne C) joue un rôle essentiel pour l'ancrage de l'orthèse sur l'avant-pied. C'est donc cette mesure qui est prise en considération pour le choix de la taille de l'orthèse, complétée par la pointure du pied. La circonférence C est mesurée en prenant comme repère les points les plus éloignés entre la tête des métatarsiens au niveau du premier métatarsien et la tête du cinquième métatarsien. On notera aussi que la longueur (en centimètres) mesurée de la racine (A) du premier orteil passant par la face interne du pied, par l'arrière du talon et revenant sur la face externe du pied jusqu'à la racine (B) du cinquième orteil. Cette mesure (AB) servira à déterminer la taille de la pointure souhaitée pour l'orthèse.

Avantageusement, l'orthèse de l'invention sous toutes ses variantes peut être réalisée en tailles allant de la taille pédiatrique jusqu'aux tailles adultes, en paires droites et gauches.

Dans ce premier exemple de réalisation, le collier métatarso- phalangien 2 se présente sous la forme d'une bande dont les portions extrêmes sont équipées d'éléments d'attache auto-agrippants à crochets et à boucles permettant à ladite bande d'être refermée sur elle-même selon une longueur réglable, ce qui permet d'ajuster la dimension du collier en fonction de la zone circonférentielle C effective de l'utilisateur.

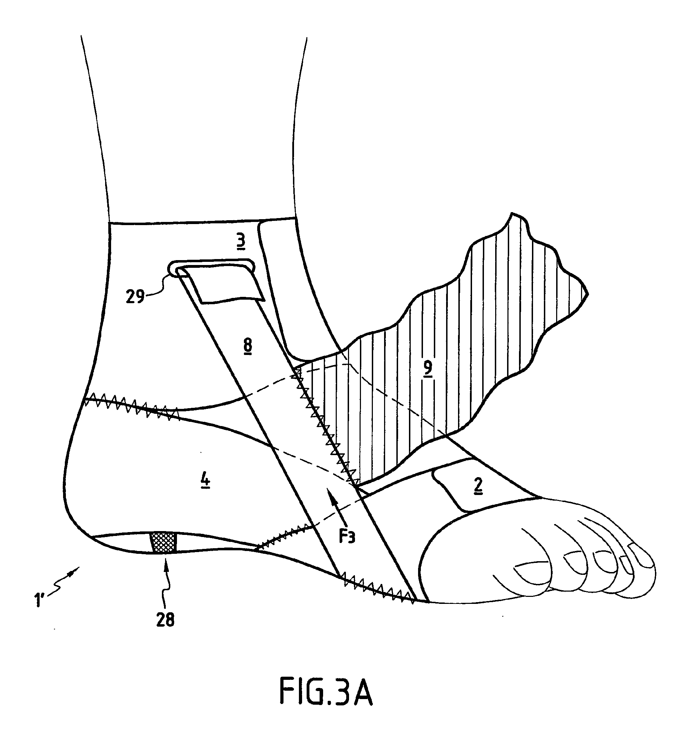

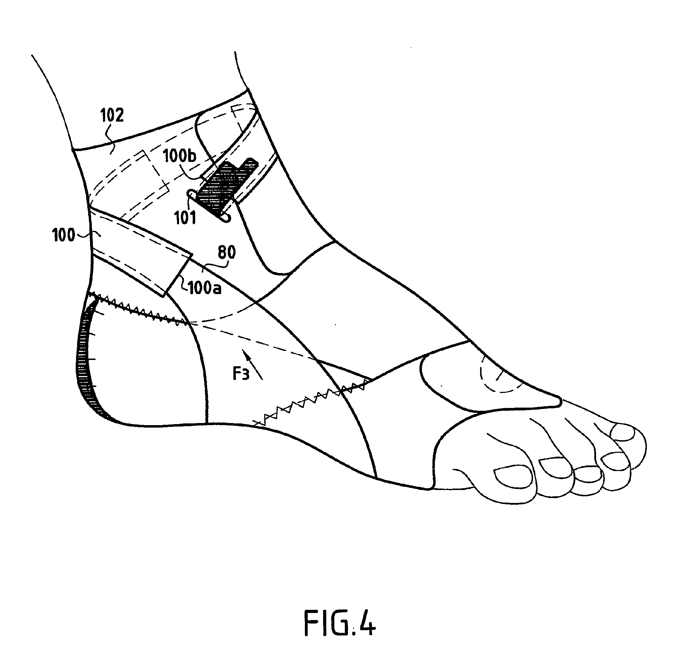

Les figures 3a à 3d illustrent un second exemple de réalisation d'une orthèse 1' qui comprend en outre une sangle de rappel élastique métatarso-fibullaire 8 et un troisième collier du tarse 9, solidaires entre eux à 90° par une ligne de couture à une distance telle que le troisième collier du tarse 9 entoure uniquement le médio-pied du patient. Dans l'exemple de réalisation de la figure 4, le collier du tarse 90 est cousu sur le bord supéro-interne de la sangle de torsion 40.

La sangle de rappel 8 a pour fonction de ramener le bord externe du pied en angle droit et d'incliner le pied par rapport à la jambe (position valgus, figure 1b, force F3). Elle est fixée, par une des ses extrémités, au premier collier métatarso-phalangien 2 par une ligne de couture 30 et elle prend appui sur le bord externe dudit premier collier métatarso-phalangien 2. Son autre extrémité vient s'accrocher par auto-agrippant sur le versant fibulaire du collier malléolaire 3, ou passe à travers une anse 29 (figure 3a) et revient sur elle-même se fixer par auto-agrippant. Dans l'exemple de réalisation de la figure 4 , la sangle de rappel 80 passe dans une ganse 100 qui fait le tour par l'arrière du tendon d'achille et vient se fixer à l'aide d'une anse 101 en avant du collier malléolaire 102. Cette ganse 100 est cousue sur la face postérieure du collier malléolaire 102 (figure 4) et angulée de sorte que son extrémité externe 100a se situe au niveau de la malléole externe et son extrémité interne 100b se situe au niveau du bord supérieur du collier malléolaire 102. L'action de la sangle de rappel 8 est illustrée sur les figures 3a et 4. La force F3 soulève la partie externe du pied puis l'ensemble de l'avant-pied, suivant la tension qui est donnée à la pose. L'axe plantaire du pied est à angle droit par rapport à l'axe fibulaire de la jambe.

Pour permettre à cette sangle de rappel 8 d'épouser parfaitement l'anatomie du trajet qu'elle suit sur le pied et le bord externe de la cheville, elle est plaquée par le troisième collier du tarse 9, qui se présente sous la forme d'une bande qui enveloppe le médio-pied et revient s'appliquer sur elle-même au delà de la ligne de couture 31 en se refermant grâce à des éléments d'attache auto-agrippants.

Dans l'exemple de réalisation illustré à la figure 3A, le troisième collier du tarse 9 est fixé par couture à la sangle de rappel 8. Ce troisième collier 9 pourrait aussi être fixé sur le bord interne antéro-supérieur de la sangle de traction 4 (non visible sur la figure 3A). De même la sangle rappel 8 pourrait avoir une configuration trapézoïdale, dont la grande base serait fixée par une ligne de couture 30 s'étendant sur toute la longueur (prise dans la direction longitudinale du pied) du premier collier métatarso-phalangien 2 et sur une partie de la longueur de la sangle de traction 4.

Enfin le second collier malléolaire 3 pourrait monter sur toute la hauteur de la cheville et même au-delà ; étant éventuellement décomposée en plusieurs tronçons de bandes, refermables sur elles-mêmes par auto agrippant. De préférence dans ce cas, le tronçon de bande supérieure présente intérieurement un matériau anti-glisse pour améliorer le blocage en position de ce second collier malléolaire 2.

L'homme du métier comprend l'importance du choix des bonnes angulations des colliers et sangles, les uns par rapport aux autres, pour que les forces bien orientées soulagent le patient et notamment favorisent la cicatrisation des ligaments.

Pour que l'orthèse 1 ou 1' soit portée régulièrement, elle doit être efficace et confortable. Le confort est obtenu par le choix des matières textiles et de matériaux de capitonnage. Les tensions appliquées sur les sangles imposent de protéger la peau dans certaines zones bien précises. C'est le cas pour la sangle de traction 4 qui, tel qu'illustré dans la figure 2, est recouverte sur sa face intérieure au niveau du talon d'un matériau isolant 11, par exemple une couche de néoprène ou silicone de faible épaisseur, rapportée localement sur la sangle 4. L'orthèse 1 évite, par sa forme de talonnière au niveau du calcanéum, la tendinite du tendon achilléen. Une pièce de capitonnage 10 de forme adéquate peut être appliquée sur la partie interne du premier collier métatarso-phalangien 2 ; cette pièce, par exemple en néoprène ou silicone, protège des gênes sous-jacentes au niveau du gros orteil (figure 2).

La surface interne du premier collier métatarso-phalangien 2 est de préférence recouverte d'une couche d'un matériau anti-glisse à fort coefficient de frottement, par exemple silicone ou néoprène, pour lui éviter de glisser vers le médio-pied du fait de la force F2 exercée par la sangle de traction 4 ou pour lui éviter de pivoter du fait de la force F3 excentrique de la sangle de rappel 8. Le premier collier 2 peut par exemple être réalisé à partir d'un tricot jersey en polyamide/lycra de forte élasticité longitudinale, contrecollé sur une couche de néoprène faisant de l'ordre de 1,5 à 2,5 mm d'épaisseur, ou entièrement réalisé en néoprène ou autre matériau. Lors de la mise en place, sous tension, du premier collier 2, celui-ci se déforme et vient épouser les contours du pied et en particulier la protubérance formée localement par les têtes des premier et cinquième métatarsiens. Cette application sous tension, combinée à l'effet anti-glisse du néoprène assure l'ancrage recherché de l'orthèse au niveau de l'avant-pied, apte à résister à la force F2 de la sangle de traction et éventuellement de la force F3 de la sangle de rappel.

Le rôle de chaque constituant de l'orthèse selon l'invention dans la thérapeutique de l'entorse du médio-pied est indiqué ci-dessous :
- la sangle de traction 4 (élastique en traction) et le premier collier métatarso-phalangien 2 produisent un rapprochement de l'avant-pied et de l'arrière-pied en prenant le médio-pied en étau (maintien axial longitudinal), rapprochant ainsi les os du tarse et les ligaments distendus ou rompus, et ayant pour conséquence la diminution voire disparition de la douleur et le rapprochement des extrémités distendues ou rompues des ligaments du médio-pied externe (articulation de Chopart et Lisfranc) ; éventuellement un anti-dérapant placé sur la face interne de la sangle de traction 4 empêche son glissement ;
- le troisième collier du tarse 9 maintien en position les os du tarse dans l'axe transversal ;
- la sangle de rappel élastique 8 prévient la chute de l'avant-pied en flexion plantaire et varus et ramène le pied en flexion dorsale et valgus, selon la phase de guérison ressentie par le patient, en soulageant la contracture des muscles péroniers ;
- la contention du pied apporte un effet anti-oedémateux.

Le concept décrit ci-dessus peut également être réalisé avec une orthèse dont les fonctions sont identiques mais la réalisation industrielle différente, bénéficiant des évolutions technologiques très récentes de la machine à tricoter rectiligne avec la commande informatisée de toutes les fonctions.

Dans cette variante de réalisation, l'orthèse de l'invention enveloppe totalement la cheville et le pied grâce à une structure tricotée en forme de chaussette. Ceci présente l'intérêt de pouvoir intégrer deux pièces rigides dans le sens de la hauteur de façon à stabiliser la cheville dans le plan frontal et éviter le varus du pied. Elle est particulièrement recommandée dans le cas de double entorse, lorsque l'entorse de la cheville s'accompagne d'une entorse du médio-pied qui pourrait passer inaperçue et donc non traitée.

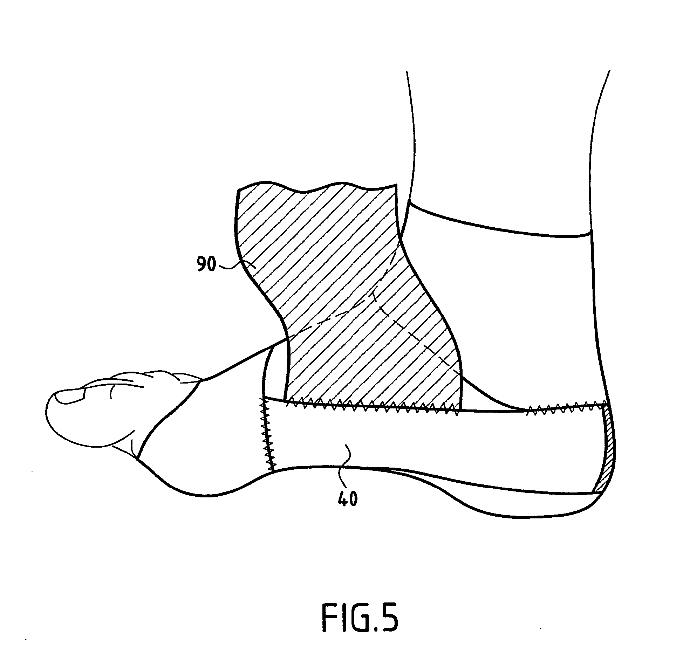

On comprend même l'intérêt de cette variante de l'invention à la vue de la figure 5 illustrant un troisième exemple de réalisation.

L'orthèse 1" selon l'invention est constituée d'une pièce en tricot 12 refermée sur elle-même à l'aide d'une couture plate 13. Le volume du talon et la courbure naturelle jambe/pied sont façonnées par la gestion informatisée du nombre de mailles à chaque rangée de tricotage de façon à obtenir un tricot trois dimensions.

La contention de l'orthèse est donnée par la présence suivant le procédé bien connu d'un fil élastique maillé ou vanisé à chaque rangée de tricotage et par la présence d'un fil élastique tramé toutes les deux rangées de tricotage sauf dans la zone du tarse 14 de façon à exercer une pression supérieure et résorber l'oedème.

L'orthèse 1" inclut un premier collier métatarso-phalangien 15 avec un matériau à fort coefficient de friction , cousu ou enduit à l'intérieur du tricot au niveau de l'avant-pied. Ce premier collier 15 bénéficie de la forme anatomique de l'avant-pied (circonférence métatarsienne la plus large).

La force de contention du tricot élastique est nécessairement complétée par une sangle ou un étrier de traction comme décrit dans le premier exemple ci-dessus. Sur la figure 5, on a représenté une sangle de traction 16 disposée sur la face externe.

Deux fourreaux 17 (un situé du côté externe et l'autre du côté interne du pied) réalisés de préférence en tissu astrakan sont cousus sur la partie supérieure de l'orthèse 1" qui entoure la cheville et qui forme un second collier malléolaire 12a ; ils permettent l'introduction, le positionnement et le maintien de deux pièces rigides 18 capitonnées par de la mousse pour le confort et la protection de la peau. Les pièces rigides s'étendent depuis la semelle du pied sur une hauteur de 12 centimètres ou plus au dessus de la pointe de la malléole fibulaire. Des bandes de fixation 24, de préférence deux du côté interne et deux du côté externe de la cheville, ayant pour origine respectivement la zone postérieure et la zone inférieure du talon, entourent le cou du pied vers l'avant pour s'attacher sur un auto-agrippant 25. Les bandes de fixation 24 servent à stabiliser les extrémités inférieures des pièces rigides 18 sous les malléoles.

Ces pièces rigides 18, en coupe transversale, sont conformées pour épouser la forme anatomique de la zone jambe/cheville/malléole, le fourreau extérieur 17 servant de surface d'accroche pour l'extrémité supérieure de la sangle élastique de rappel 19.

Cette sangle de rappel 19 de très forte contention est fixée par une couture 20 au premier collier métatarso-phalangien 15, avec la bonne angulation comme expliqué plus haut. Elle soulève la partie externe du pied puis l'ensemble de l'avant-pied suivant la tension qui est donnée à la pièce, selon la phase de guérison ressentie par le patient.

Cette sangle 19 de rappel est équipée d'un système auto-agrippant par exemple à crochets et à boucles et vient se fixer par son extrémité supérieure sur la face extérieure du fourreau 17, ou passe à travers une anse 27 et revient sur elle-même se fixer par auto-agrippant 26.

Afin de faciliter la pose de l'orthèse 1", la chaussette de contention 12 est équipée à l'extrémité supérieure d'une fermeture à glissière 23. Elle se substitue à la couture d'assemblage 13 sur une hauteur allant du niveau des malléoles à l'extrémité supérieure de la chaussette.

L'orthèse peut comporter une ganse (21) servant à solidariser la sangle de rappel (19) à la pièce tricotée (12).

Une bande de fixation 22, fixée sur l'arrière de la chaussette 12, se referme sur elle-même au-dessus des malléoles et évite tout déplacement des pièces rigides ainsi que le décrochage de la sangle 19.

Dans les modes de réalisation décrits plus haut, les différentes sangles entrant dans la constitution de l'orthèse selon l'invention : les sangles de rappel 8, 19 ainsi que les bandes de fixation 24, sont disposées sur la face externe de l'orthèse 1' et 1" respectivement. Ces sangles peuvent aussi être disposées sur la face interne de l'orthèse 1' ou 1", auquel cas elles sont recouvertes du côté interne d'une couche de matériau textile adapté à un contact direct avec la peau.

L'orthèse selon l'invention procure aux patients présentant une atteinte du médio-pied et/éventuellement de la cheville de nombreux avantages. Le port de cette orthèse, facile à mettre et à enlever, favorise son utilisation par le patient, lui apporte une sécurité dans le mouvement et l'incite à une reprise de la marche, début précoce de la rééducation. Son action d'étau du médio-pied soulage la douleur, favorise la cicatrisation et permet au pied de résister efficacement aux sollicitations extérieures limitant considérablement le risque de récidive. La contention du pied assurée par les différents éléments élastiques qui composent l'orthèse favorise le drainage de l'oedème.

## Revendications

1. Orthèse souple amovible pour atteinte du médio-pied **caractérisée en ce qu'**elle comporte :
a) un premier collier (2, 15) et
b) au moins une sangle de traction (4,16) dont au moins une extrémité est fixée ou fixable sur le premier collier métatarso-phalangien (2,15), ladite sangle de traction s'étendant entre ledit premier collier (2,15) et la partie de l'orthèse destinée à être appliquée sur l'arrière du pied **caractérisé en ce que** le premier collier (2,15) est un collier métatarso-phalangien apte à être fixé sur l'avant-pied, s'étendant, transversalement, du pied des têtes du premier et cinquième métatarses et, longitudinalement, depuis l'arrière de la racine des orteils vers la mi-diaphyse des métatarses

2. Orthèse souple selon la revendication 1 **caractérisée en ce que** la sangle de traction (4) a une forme d'étrier métatarso-calcanéo-métatarsien, ses deux extrémités étant fixées sur les bords latéraux postéro-externe et postéro-interne du premier collier métatarso-phalangien (2).

3. Orthèse selon l'une des revendications 1 ou 2 **caractérisée en ce que** le premier collier métatarso-phalangien comporte sur sa face intérieure un matériau anti-glisse, à coefficient de frottement élevé, formé par une couche de néoprène.

4. Orthèse souple selon l'une des revendications 1 à 3 **caractérisée en ce qu'**elle comporte un second collier malléolaire (3,12a) apte à être fixé autour de la cheville et qui est, au moins en partie, fixé à la sangle de traction (4,16).

5. Orthèse souple selon l'une des revendications 1 à 4 **caractérisée en ce qu'**elle comporte :
- une sangle élastique de rappel (8) s'étendant entre le premier collier métatarso-phalangien (2) et le second collier malléolaire (3) et
- un troisième collier du tarse (9) destiné à recouvrir les os du tarse et à se refermer sur lui-même en s'appliquant sur la sangle de rappel (8), fixé à la sangle de rappel (8) ou fixé sur le bord supéro-interne de la sangle de traction (4).

6. Orthèse souple selon la revendication 5 **caractérisée en ce que** la sangle de rappel (8) est fixée sur le premier collier (2) selon une ligne de fixation correspondant au bord externe du pied.

7. Orthèse souple selon les revendications 4 et 5 **caractérisée en ce qu'**elle forme un ensemble cohérent dans lequel :
- la sangle de traction (4) est cousue d'une part au premier collier métatarso-phalangien (2) et d'autre part au second collier malléolaire (3) et
- la sangle de rappel (8) est cousue au premier collier (2) métatarso-phalangien et est fixée au second collier malléolaire (3) grâce à un système d'attache auto-agrippant à crochets et à boucles.

8. Orthèse selon la revendication 7 **caractérisée en ce que** la sangle de rappel est cousue au collier du tarse (9).

9. Orthèse selon la revendication 7 **caractérisée en ce que** la sangle de rappel (80) est cousue au premier collier métatarso-phalangien et est fixée au collier malléolaire (102) en passant dans une ganse (100) située sur le collier malléolaire (102) sur sa face postérieure, venant se fixer en avant du collier malléolaire (102) grâce à un système d'attache auto-agrippant à crochets et à boucles en passant dans une anse, ladite sangle (80) pouvant être unique latérale externe, ou bilatérale symétrique.

10. Orthèse souple selon l'une quelconque des revendications 1 à 6, comportant une pièce tricotée (12) en forme de chaussette refermée sur elle-même à l'aide d'une couture (13) ou d'une couture et d'une fermeture à glissière (23).

11. Orthèse souple selon la revendication 10 **caractérisée en ce qu'**elle comporte deux fourreaux (17) interne et externe, fixés sur la partie de la pièce tricotée qui fait office de second collier malléolaire (12a), pour le placement de plaques rigides, le fourreau externe (17) servant de surface d'accroche pour l'extrémité de la sangle élastique de rappel (19).

12. Orthèse selon l'une quelconque des revendications 10 et 11 **caractérisée en ce qu'**elle comporte une ganse (21) servant à solidariser la sangle de rappel (19) à la pièce tricotée (12).

13. Orthèse selon l'une quelconque des revendications 11 ou 12 **caractérisée en ce qu'**elle comporte des bandes de fixation (24) du côté interne et du côté externe de la cheville, ayant pour origine l'arrière et le dessous du talon et étant destinées à stabiliser les extrémités inférieures des plaques rigides contre les malléoles.

14. Orthèse souple selon l'une des revendications 5 à 13 **caractérisée en ce que** la sangle de rappel (8, 19) est cousue sur le premier collier métatarso-phalangien (2,15) et est apte à être fixée sur le second collier malléolaire (3,12a) grâce à un système d'attache auto-agrippant à crochets et à boucles.

15. Orthèse souple selon l'une des revendications 13 ou 14 **caractérisée en ce que** la sangle de rappel (8,19) et les bandes de fixation (24) sont disposées soit sur la face extérieure soit sur la face intérieure de l'orthèse.

## Claims

1. A removable flexible orthosis for injuries to the midfoot, which includes:
a) a first collar (2, 15), and
b) at least one traction strap (4, 16), of which at least one end is fixed or fixable on the first collar (2, 15), with the said traction strap extending between the first collar and the part of the orthesis intended to be applied to the rear of the foot,
**characterised in that** the first collar (2, 15) is a metatarsophalangeal collar (2, 15) designed to be fitted to the forefoot, extending, transversally, from the root of the heads of the first and fifth metatarsals and, longitudinally, from the rear of the toe roots to the mid-diaphysis of the metatarsals.

2. A flexible orthosis according to claim 1, **characterised in that** the traction strap (4) has the shape of a metatarso-calcaneo-metatatarsial stirrup, with its two ends being fixed on the lateral rear outer and rear inner edges of the first metatarsophalangeal collar (2).

3. An orthosis according to either of claims 1 or 2, **characterised in that** the first metatarsophalangeal collar includes, on its inner face, a non-slip material with a high friction coefficient.

4. A flexible orthosis according to one of claims 1 to 3, **characterised in that** it includes a second malleolar collar (3, 12a) designed to be placed around the ankle, and which is, at least in part, fixed to the traction strap (4, 16).

5. A flexible orthosis according to one of claims 1 to 4, **characterised in that** it includes:
- an elastic return strap (8) extending between the first metatarsophalangeal collar (2) and the second malleolar collar (3) and
- a third collar on the tarsus (9) intended to cover the bones of the tarsus and to close onto itself by being applied onto the return strap (8), fixed to the return strap (8), or fitted to the top inside edge of the traction strap (4).

6. A flexible orthosis according to claim 5, **characterised in that** the return strap (8) is fixed to the first collar (2) along an attachment line corresponding to the outer edge of the foot.

7. A flexible orthosis according to claims 4 and 5, **characterised in that** it forms a coherent assembly in which
- the traction strap (4) is sewn firstly to the first metatarsophalangeal collar (2) and secondly to the second malleolar collar (3) and
- the return strap (8) is sewn to the first metatarsophalangeal collar (2) and is attached to the second malleolar collar (3) by means of a self-stick attaching system.

8. An orthosis according to claim 7, **characterised in that** the return strap is sewn to the collar of the tarsus (9).

9. An orthosis according to claim 7, **characterised in that** the return strap (80) is sewn to the first metatarsophalangeal collar and is fixed to the malleolar collar (102) by passing through a loop (100) located on the malleolar collar (102), on its rear face, then being fixed onto the front of the malleolar collar (102) by means of a self-stick attaching system, by passing through a loop, with the said strap (80) capable of being of the single lateral outer or bilateral symmetrical type.

10. A flexible orthosis according to any of claims 1 to 6, that includes a knitted element (12) in the form of a sock that is closed onto itself by means of stitching (13) or of stitching and a zip fastener (23).

11. A flexible orthosis according to claim 10, **characterised in that** it includes two ducts (17), inner and outer, fixed onto the part of the knitted element that acts as a second malleolar collar (12a), for the placement of rigid boards, with the outer duct (17) acting as an attachment surface for the end of the elastic return strap (19).

12. An orthosis according to either of claims 10 and 11, **characterised in that** it includes a loop (21) that is used to attach the return strap (19) to the knitted element (12).

13. An orthosis according to either of claims 11 or 12, **characterised in that** it includes attachment straps (24) on the inside and on the outside of the ankle, having as their origin the rear and the bottom of the heel, and intended to stabilise the bottom ends of the rigid boards against the malleoli.

14. A flexible orthosis according to one of claims 5 to 13, **characterised in that** the return strap (8, 19) is sewn to the first metatarsophalangeal collar (2, 15), and is designed to be fixed on the second malleolar collar (3, 12a) by means of a self-attaching system of the hook and loop (velcro) type.

15. A flexible orthosis according to either of claims 13 or 14, **characterised in that** the return strap (8, 19) and the attachment straps (24) are positioned either on the outer face or on the inner face of the orthosis.

## Patentansprüche

1. Abnehmbare flexible Orthese für Mittelfußverletzungen, die folgendes umfaßt:
a) ein erstes Band (2, 15) und
b) wenigstens einen Zuggurt (4, 16), von dem wenigstens ein Ende fest ist oder an dem ersten Band befestigt werden kann, wobei der Zuggurt zwischen dem ersten Band und dem Teil der Orthese verläuft, der dazu bestimmt ist, am hinteren Teil des Fußes angelegt zu werden,
**dadurch gekennzeichnet, daß** das erste Band (2, 15) ein Metatarsophalangeal-Band ist, das geeignet ist, am Vorfuß befestigt zu werden, sich quer von der Basis der Köpfchen des ersten und des fünften Mittelfußknochens aus erstreckt sowie in Längsrichtung vom hinteren Teil der Wurzel der Zehen zur Mitte der Diaphyse der Mittelfußknochen verläuft.

2. Flexible Orthese nach Anspruch 1, **dadurch gekennzeichnet, daß** der Zuggurt (4) die Form eines Mittelfuß-Fersenbein-Mittelfuß-Bügels aufweist, wobei seine beiden Enden an den hinteren äußeren und hinteren inneren Seitenrändern des ersten Metatarsophalangeal-Bandes (2) befestigt sind.

3. Orthese nach einem der Ansprüche 1 oder 2, **dadurch gekennzeichnet, daß** das erste Metatarsophalangeal-Band an seiner Innenseite ein rutschhemmendes Material mit hohem Reibbeiwert aufweist.

4. Flexible Orthese nach einem der Ansprüche 1 bis 3, **dadurch gekennzeichnet, daß** sie ein zweites Malleolar-Band (3, 12a) umfaßt, das geeignet ist, um den Knöchel herum befestigt zu werden und das wenigstens teilweise an dem Zuggurt (4, 16) befestigt ist.

5. Flexible Orthese nach einem der Ansprüche 1 bis 4, **dadurch gekennzeichnet, daß** sie folgendes umfaßt:
- einen elastischen Rückstellgurt (8), der zwischen dem ersten Metatarsophalangeal-Band (2) und dem zweiten Malleolar-Band (3) verläuft, und
- ein drittes Fußwurzel-Band (9), das dazu bestimmt ist, die Knochen der Fußwurzel zu bedecken und - sich über den Rückstellgurt (8) legend - an sich selbst verschlossen zu werden, an dem Rückstellgurt (8) befestigt oder an dem oberen inneren Rand des Zuggurtes (4) befestigt ist.

6. Flexible Orthese nach Anspruch 5, **dadurch gekennzeichnet, daß** der Rückstellgurt (8) an dem ersten Band (2) entlang einer dem Außenrand des Fußes entsprechenden Befestigungslinie befestigt ist.

7. Flexible Orthese nach den Ansprüchen 4 und 5, **dadurch gekennzeichnet, daß** sie eine kohärente Einheit bildet, bei der:
- der Zuggurt (4) einerseits an dem ersten Metatarsophalangeal-Band (2) und andererseits an dem zweiten Malleolar-Band (3) angenäht ist, und
- der Rückstellgurt (8) an dem ersten Metatarsophalangeal-Band (2) angenäht und an dem zweiten Malleolar-Band (3) mittels eines selbsthaftenden Befestigungssystems befestigt ist.

8. Orthese nach Anspruch 7, **dadurch gekennzeichnet, daß** der Rückstellgurt an dem Fußwurzel-Band (9) angenäht ist.

9. Orthese nach Anspruch 7, **dadurch gekennzeichnet, daß** der Rückstellgurt (80) an dem ersten Metatarsophalangeal-Band angenäht und unter Durchlaufen einer an dem Malleolar-Band (102), an dessen Rückseite befindlichen Schlaufe (100) an dem Malleolar-Band (102) befestigt ist, wobei er vorne an dem Malleolar-Band (102) unter Durchlaufen einer Öse mittels eines selbsthaftenden Befestigungssystems festgelegt ist, wobei der Gurt (80) lediglich einseitig außen oder zweiseitig symmetrisch sein kann.

10. Flexible Orthese nach einem der Ansprüche 1 bis 6, umfassend ein sockenförmiges Strickteil (12), das mittels einer Naht (13) oder einer Naht und einem Reißverschluß (23) an sich selbst verschlossen ist.

11. Flexible Orthese nach Anspruch 10, **dadurch gekennzeichnet, daß** sie zwei Hülsen (17), eine innere und eine äußere, für das Anbringen starrer Platten umfaßt, die an dem Teil des Strickteils befestigt sind, der als zweites Malleolar-Band (12a) dient, wobei die äußere Hülse (17) als Befestigungsfläche für das Ende des elastischen Rückstellgurts (19) dient.

12. Orthese nach einem der Ansprüche 10 und 11, **dadurch gekennzeichnet, daß** sie eine Schlaufe (21) umfaßt, die dazu dient, den Rückstellgurt (19) mit dem Strickteil (12) fest zu verbinden.

13. Orthese nach einem der Ansprüche 11 oder 12, **dadurch gekennzeichnet, daß** sie Befestigungsbänder (24) auf der Innenseite und auf der Außenseite des Knöchels umfaßt, die vom hinteren Teil und der Unterseite der Ferse ausgehen und die dazu bestimmt sind, die unteren Enden der starren Platten an den Knöcheln zu stabilisieren.

14. Flexible Orthese nach einem der Ansprüche 5 bis 13, **dadurch gekennzeichnet, daß** der Rückstellgurt (8, 19) an dem ersten Metatarsophalangeal-Band (2, 15) angenäht und geeignet ist, mittels eines selbsthaftenden Befestigungssystems mit Häkchen und Schlingen an dem zweiten Malleolar-Band (3, 12a) befestigt zu werden.

15. Flexible Orthese nach einem der Ansprüche 13 oder 14, **dadurch gekennzeichnet, daß** der Rückstellgurt (8, 19) und die Befestigungsbänder (24) entweder an der Außenseite oder an der Innenseite der Orthese angeordnet sind.
